# EUROPEAN PATENT APPLICATION

(11) **EP 2 128 150 A1**
(43) Date of publication of application: **02.12.2009**
(21) Application number: 07832707.9
(22) Date of filing: 28.11.2007
(51) Int. Cl.: C07D 317/42, C08F 16/38

(54) **FLUORINE-CONTAINING COMPOUND AND FLUORINE-CONTAINING POLYMER**

(30) Priority: 20.12.2006 JP 2006343518
(71) Applicant: Asahi Glass Company, Limited, Chiyoda-ku Tokyo 100-8405 (JP)
(72) Inventor: MATSUURA, Keigo, Tokyo 100-8405 (JP); SUGIYAMA, Norihide, Tokyo 100-8405 (JP)
(74) Representative: Müller-Boré & Partner Patentanwälte
(86) International application number: PCT/JP2007/072985
(87) International publication number: WO 2008/075545

(57) **Abstract**

The present invention is to provide a novel fluorinated compound which can produce a fluorinated polymer which has high transparency, good heat resistance and adequate flexibility, and a fluorinated polymer containing repeating units based on the fluorinated compound. The present invention relates to a fluorinated compound represented by the formula (1): 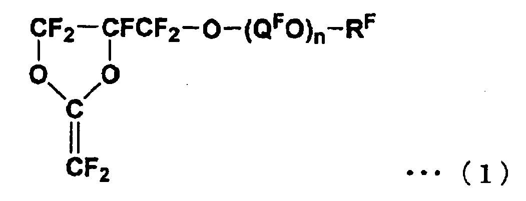 wherein Q^{F} is -CF₂-, -CF₂CF₂-, -CF₂CF₂CF₂-, -CF(CF₃)CF₂-, -CF₂CF(CF₃)- or -CF₂CF₂CF₂CF₂-, R^{F} is -CF₃, -CF₂CF₃, -CF₂CF₂CF₃, -CF(CF₃)₂, -CF₂CF₂CF₂CF₃, -CF₂CF(CF₃)₂, -CF(CF₃)CF₂CF₃, -(CF₂)₄CF₃ or -(CF₂)₅CF₃, and n is an integer of from 1 to 4, and a fluorinated polymer containing repeating units based on the fluorinated compound.

## Description

### TECHNICAL FIELD

The present invention relates to a novel fluorinated dioxolane compound which can be a raw material for a fluorinated polymer excellent in physical properties such as heat resistance and transparency, and a fluorinated polymer comprising repeating units based on the fluorinated dioxolane compound.

### BACKGROUND ART

A fluorinated polymer has been employed for, for example, an optical material such as a plastic optic fiber, or a sealing material for a device such as a semiconductor device, and is a useful substance to be used in various technical fields.

The fluorinated polymer is obtained by polymerizing a fluorinated compound having a polymerizable unsaturated group. As such a fluorinated compound, a compound represented by the following formula (I) has, for example, been disclosed: 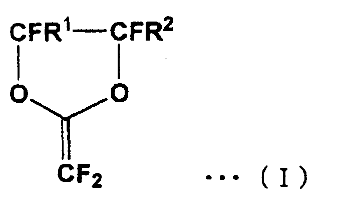 wherein each of R¹ and R² which are the same or different from each other, represents a C₁₋₇ polyfluoroalkyl group, or R¹ and R² represent a connected C₂₋₅ polyfluoroalkylene group (Patent Document 1).

Patent Document 1 discloses that the compound represented by the above formula (I) is polymerized alone or copolymerized with another monomer to obtain a fluorinated polymer soluble in a special solvent, and the fluorinated polymer can be applied to a low reflective coating, a thin film or the like.

Further, as such a fluorinated compound, a compound represented by the following formula (II) has, for example, been disclosed: 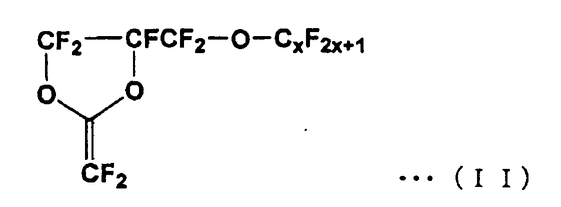 wherein x represents an integer of from 1 to 3 (Patent Document 2).

The Patent Document 2 discloses that the compound represented by the above formula (II) is polymerized alone or copolymerized with another monomer to obtain a fluorinated polymer which has a high glass transition temperature and is amorphous and excellent in heat resistance, light resistance, etc.

Each of the fluorinated compounds represented by the formula (I) and the formula (II) is subjected to bulk polymerization to obtain a fluorinated polymer having high transparency and stiffness.

### DISCLOSURE OF THE INVENTION

### OBJECT TO BE ACCOMPLISHED BY THE INVENTION

However, the fluorinated polymer obtained by subjecting each of the fluorinated compounds represented by the formula (I) and the formula (II) to bulk polymerization was fragile and had a problem that cracking occurred during the bulk polymerization.

In order to avoid the cracking during the bulk polymerization, it is necessary for the polymer to have adequate flexibility, but heretofore, a fluorinated polymer having high transparency and flexibility has not been obtained.

Therefore, the present invention is to provide a novel fluorinated compound which can produce a fluorinated polymer which has high transparency, good heat resistance and adequate flexibility, and a fluorinated polymer containing repeating units based on the fluorinated compound.

### MEANS TO ACCOMPLISH THE OBJECT

The present invention provides the following:
[1] A fluorinated compound represented by the following formula (1): 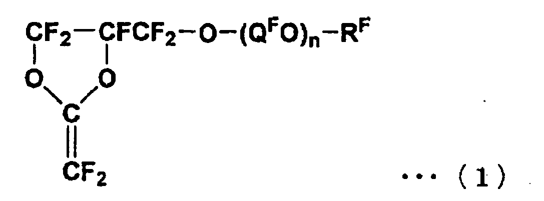 provided that, in the formula (1), Q^{F} is -CF₂-, -CF₂CF₂-, -CF₂CF₂CF₂-, -CF(CF₃)CF₂-, -CF₂CF(CF₃)-or -CF₂CF₂CF₂CF₂-, R^{F} is -CF₃, -CF₂CF₃, -CF₂CF₂CF₃, -CF(CF₃)₂, -CF₂CF₂CF₂CF₃, -CF₂CF(CF₃)₂, -CF(CF₃)CF₂CF₃, -(CF₂)₄CF₃ or -(CF₂)₅CF₃, and n is an integer of from 1 to 4.
[2] The fluorinated compound according to the above [1], wherein Q^{F} in the above formula (1) is -CF₂CF₂-, -CF₂CF₂CF₂- or -CF₂CF₂CF₂CF₂-.
[3] The fluorinated compound according to the above [1] or [2], wherein n in the above formula (1) is 1 or 2.
[4] A fluorinated compound represented by one of the following formulae: 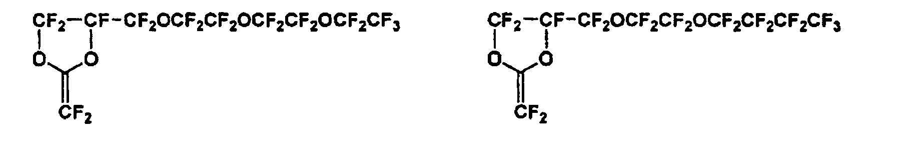 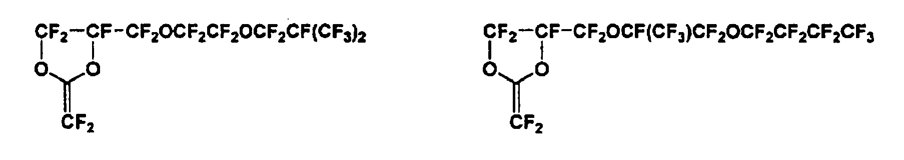 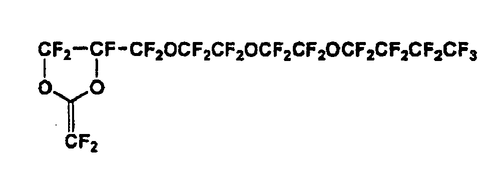
[5] A fluorinated polymer comprising repeating units represented by the following formula (2): 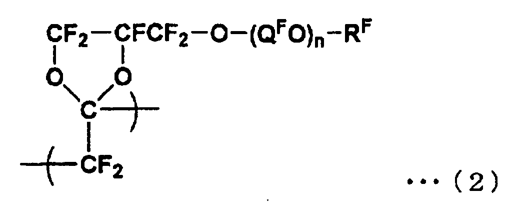 provided that, in the formula (2), Q^{F} is -CF₂-, -CF₂CF₂-, -CF₂CF₂CF₂-, -CF(CF₃)CF₂-, -CF₂CF(CF₃)- or -CF₂CF₂CF₂CF₂-, R^{F} is -CF₃, -CF₂CF₃, -CF₂CF₂CF₃, -CF(CF₃)₂, -CF₂CF₂CF₂CF₃, -CF₂CF(CF₃)₂, -CF(CF₃)CF₂CF₃, -(CF₂)₄CF₃ or -(CF₂)₅CF₃, and n is an integer of from 1 to 4.
[6] The fluorinated polymer according to the above [5], wherein Q^{F} in the above formula (2) is -CF₂CF₂-, -CF₂CF₂CF₂- or -CF₂CF₂CF₂CF₂-.
[7] The fluorinated polymer according to the above [5] or [6], wherein n in the above formula (2) is 1 or 2.
[8] The fluorinated polymer according to any one of the above [5] to [7], which contains from 30 to 100 mass% of repeating units represented by the above formula (2).
[9] The fluorinated polymer according to any one of the above [5] to [8], which contains repeating units based on another monomer, and said another monomer is a fluorinated monomer.
[10] The fluorinated polymer according to the above [9], wherein the fluorinated monomer is at least one selected from the group consisting of tetrafluoroethylene, hexafluoropropylene, chlorotrifluoroethylene, a perfluoro(alkyl vinyl ether), perfluoro(allyl vinyl ether), perfluoro(butenyl vinyl ether), perfluoro(2,2-dimethyl-1,3-dioxole), perfluoro(4-methoxy-1,3-dioxole, a perfluoro(3-alkyl-2-methylene-1 ,3-dioxolane) and a perfluoro(3-alkoxyalkyl-2-methylene-1,3-dioxolane).

### EFFECTS OF THE INVENTION

It is possible for the fluorinated compound of the present invention to provide a fluorinated polymer having high transparency, good heat resistance and adequate flexibility.

Further, the fluorinated polymer of the present invention has high transparency, good heat resistance and adequate flexibility.

### BEST MODE FOR CARRYING OUT THE INVENTION

### Fluorinated compound

The fluorinated compound of the present invention is represented by the following formula (1): 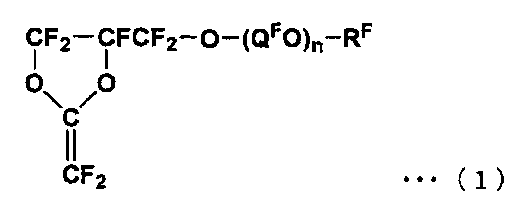 provided that, in the formula (1), Q^{F} is -CF₂-, -CF₂CF₂-, -CF₂CF₂CF₂-, -CF(CF₃)CF₂-, -CF₂CF(CF₃)- or -CF₂CF₂CF₂CF₂-, R^{F} is -CF₃, -CF₂CF₃, -CF₂CF₂CF₃, -CF(CF₃)₂, -CF₂CF₂CF₂CF₃, -CF₂CF(CF₃)₂, -CF(CF₃)CF₂CF₃, -(CF₂)₄CF₃ or -(CF₂)₅CF₃, and n is an integer of from 1 to 4.

In the present specification, the compound represented by the formula (1) is also represented by "compound (1)". The compounds represented by other formulae are also represented in the same manner.

As Q^{F} and R^{F}, preferred is (Q^{F}O)ₙ-R^{F} having a total number of carbon atoms of from 4 to 13. When the number of carbon atoms is at least 4, the boiling point suitable for bulk polymerization tends to be readily obtained, such being preferred. Further, when the number of carbon atoms is at most 13, the purification by distillation tends to be easy, such being preferred. Further, each of Q^{F} and R^{F} is preferably linear since a flexible polymer is thereby obtained. n is particularly preferably 1 or 2 since an adequate glass transition temperature is thereby obtained.

Q^{F} is particularly preferably -CF₂CF₂-, -CF₂CF₂CF₂- or -CF₂CF₂CF₂CF₂-.

R^{F} is particularly preferably -CF₃, -CF₂ CF₃, -CF₂CF₂CF₃ or -CF₂CF₂CF₂CF₃.

Q^{F}, R^{F} and n in the above formula (1) may be optionally selected from the above respective groups and combined, and a combination of the above respective preferred ones is more preferred.

As the compound (1), particularly preferred ones are exemplified below.

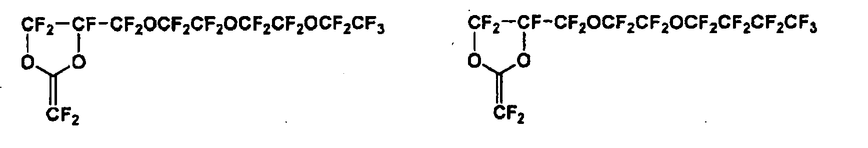

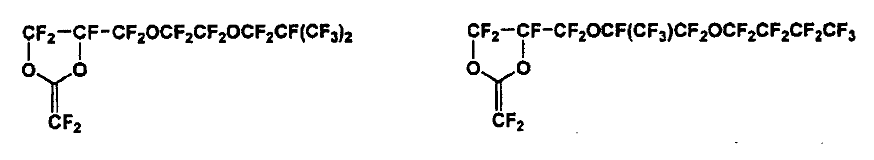

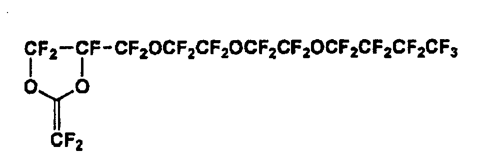

Among the above, most preferred is one wherein Q^{F} is -CF₂CF₂-, R^{F} is -CF₂ CF₂CF₂CF₃ and n is 1, since the purification by distillation is easy and a flexible polymer is obtainable.

### < Process for producing the compound (1) >

The compound (1) can be produced, for example, by the following synthetic route. However, the process for producing the compound (1) is not limited to the following synthetic route.

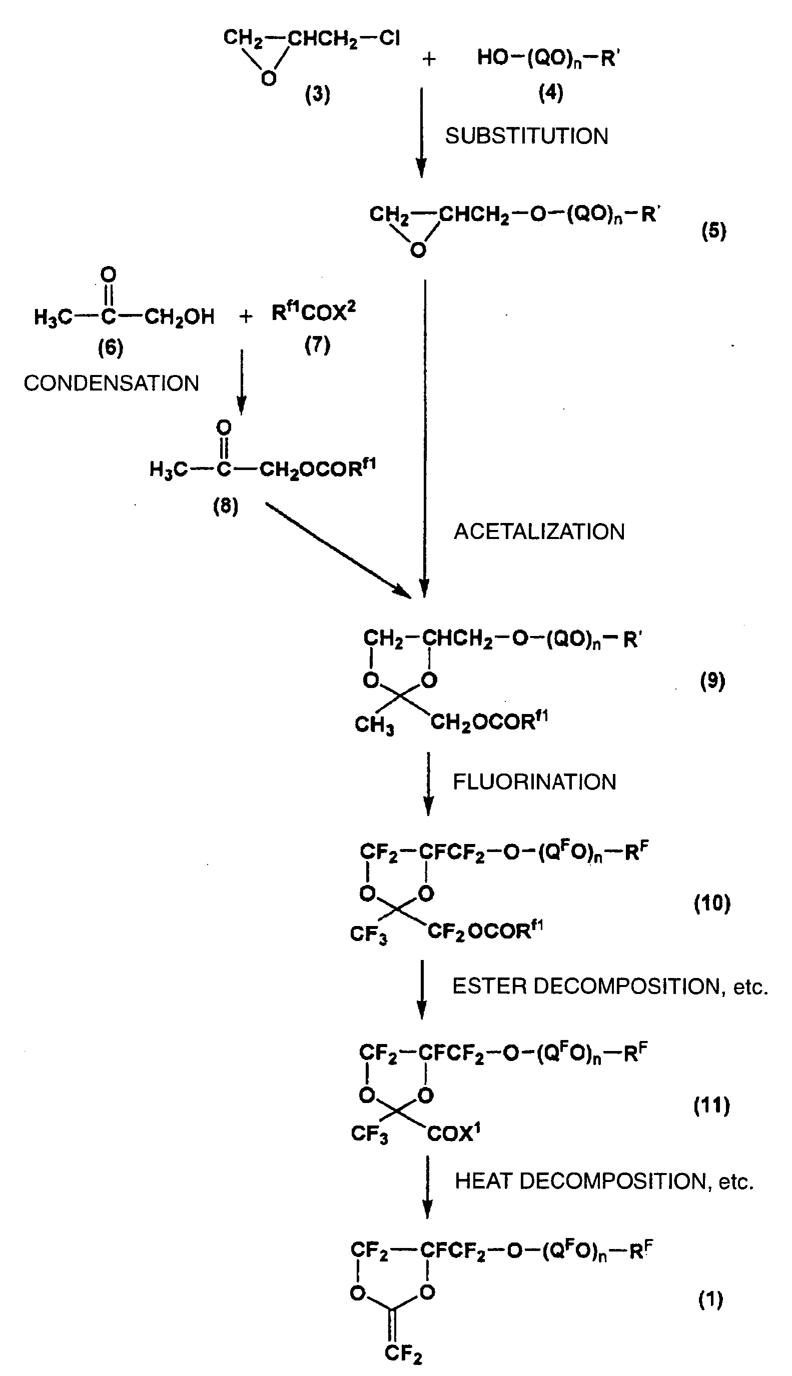

In the above synthetic route, firstly, the compound (3) (epichlorohydrin) and the compound (4) (alcohol) are subjected to a substitution reaction (condensation reaction) to obtain the compound (5).

Separately, the compound (6) (hydroxyacetone) and the compound (7) (fluorinated acyl halide) are subjected to a condensation reaction to obtain the compound (8).

Further, the compound (5) and the compound (8) are subjected to an acetalization reaction to obtain the compound (9). Then, the compound (9) is subjected to a fluorination (perfluorination) reaction to obtain the compound (10).

Further, e.g. a decomposition reaction of an ester bond in the compound (10) is conducted to obtain the compound (11). Then, the compound (11) is subjected to e.g. heat decomposition to obtain the compound (1).

In the above synthetic route, the group represented by "(QO)ₙ- R^{'}", wherein Q is -CH₂-, -CH₂CH₂-, -CH₂CH₂CH₂-, -CH(CH₃)CH₂-, -CH₂CH(CH₃)- or -CH₂CH₂CH₂CH₂-, R^{'} is -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -(CH₂)₄CH₃ or -(CH₂)₅CH₃ and n is an integer of from 1 to 4, corresponds to "(Q^{F}O)ₙ-R^{F}" in the above formula (1), wherein all of the fluorine atoms are changed to hydrogen atoms.

The group represented by "(Q^{F}O)ₙ-R^{F}" is the same as "(Q^{F}O)ₙ-R^{F}" in the above formula (1).

R^{f1} represents a C₁₋₁₀ fluorinated hydrocarbon or perfluoroalkyl group, provided that between carbon atom-carbon atom in the fluorinated hydrocarbon or perfluoroalkyl group, 1 to 5 etheric oxygen atoms may be inserted.

X² represents a halogen atom.

X¹ represents a halogen atom or a group represented by -OR (wherein R represents an alkali metal atom, a hydrogen atom, a C₁₋₅ alkyl group or a group having 1 or 2 etheric oxygen atoms inserted between carbon atom-carbon atom in a C₁₋₅ alkyl group, and among them, an alkyl group or a hydrogen atom is preferred, a C₁₋₄ alkyl group or a hydrogen atom is particularly preferred.).

The compounds (9), (10) and (11) are novel compounds and useful as intermediates for the production of the compound (1).

The respective reaction steps will be described below.

### [Steps until the compound (9) is obtained]

The compound (9) is obtained by subjecting the compound (5) and the compound (8) to an acetalization reaction.

The compound (5) is obtained by subjecting the compound (3) (epichlorohydrin) and the compound (4) (alcohol) to a substitution reaction (condensation reaction).

Further, the compound (8) is obtained by subjecting the compound (6) (hydroxyacetone) and the compound (7) (fluorinated acyl halide) to a condensation reaction.

In the condensation reaction between the compound (6) and the compound (7), the reaction temperature is preferably from -50°C to 100°C.

In a case where X² in the compound (7) is a fluorine atom, HF may form as a byproduct. In order to remove the byproduct, preferred is to add a HF capturing agent such as NaF or KF to the reaction system, or to blow an inert gas into the reaction system so that HF is discharged out of the reaction system as accompanied with the inert gas. Further, in a case where X² is a chlorine atom, HCl may form as a byproduct, and it is preferred to add an acid scavenger.

The acetalization reaction between the compound (5) and the compound (8) is preferably conducted in acetone as a solvent in the presence of an acid catalyst. Further, in a case where the acid catalyst is deactivated by e.g. moisture in the air, the reaction is preferably conducted under a dehydrated atmosphere.

The acid catalyst is preferably a Lewis acid, and for example, an inorganic acid such as hydrochloric acid, sulfuric acid, boron trifluoride or aluminum chloride, an organic acid such as a p-toluenesulfonic acid, or a solid acid such as an acidic ion-exchange resin may be mentioned.

With respect to the reaction temperature in the acetalization reaction, the lower limit is preferably 0°C and the upper limit is preferably the lowest boiling point among the boiling points of the compounds used in the reaction.

The acetalization reaction is an equilibrium reaction between dimethyl acetal and the compound (9), and in order to let the equilibrium shift towards the compound (9) and to remove the byproduct, it is preferred that the temperature is adjusted to higher than the boiling point of acetone or the byproduct at the end of the reaction and that acetone or the byproduct is gasified and discharged out of the reaction system.

### [Another step for obtaining the compound (9) from the compound (5)]

Another step for obtaining the compound (9) may be a process wherein the compound (5) and the compound (6) are subjected to an acetalization reaction to obtain the compound (12), followed by a condensation reaction with the compound (7).

The acetalization reaction between the compound (5) and the compound (6) can be conducted by using an acid catalyst similar to the one used for the acetalization reaction between the compound (5) and the compound (8). The condensation reaction between the compound (7) and the compound (12) may also be conducted under a condition similar to the one for the condensation reaction between the compound (6) and the compound (7).

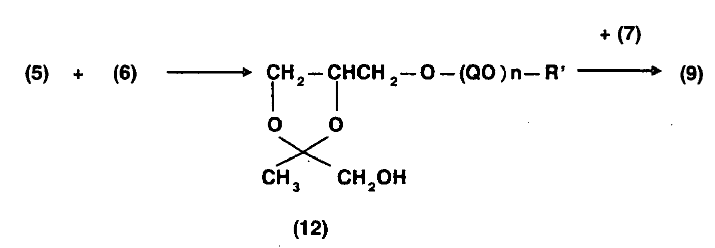

### [Step for obtaining the compound (10)]

The compound (10) is obtained by subjecting the compound (9) to a fluorination (perfluorination).

The fluorination is a reaction to replace a hydrogen atom in the compound (9) by a fluorine atom. In the present invention, the fluorination reaction is continued until all of the hydrogen atoms in the compound (9) are replaced by fluorine atoms (that is, until perfluorinated). The fluorination reaction is usually conducted in a liquid phase.

The fluorination reaction in a liquid phase (hereinafter referred to as liquid phase fluorination reaction) is preferably conducted in a solvent in accordance with a normal method. As such a solvent, preferred is one which can dissolve both the compound (9) and the compound (10) which is a byproduct produced during the liquid phase fluorination reaction. The solvent is preferably a fluorine type solvent inert to the liquid phase fluorination reaction, more preferably a solvent which can dissolve at least 1 mass% of the compound (10), particularly preferably a solvent which can solve at least 5 mass% of the compound (10). Specifically, a perfluoroalkane (for example, tradename FC-72 manufactured by 3M), a perfluoroether (for example, tradename "FC-75" or "FC-77" manufactured by 3M), a perfluoropolyether (for example, tradename "Krytox" manufactured by Du Pont, "Fomblin" or "Galden" manufactured by Ausimont Inc., tradename "Demnum" manufactured by Daikin Industries, Ltd.), a chlorofluorocarbon or a perfluoroalkylamine (for example, tradename "FC-43" manufactured by 3M) may, for example, be mentioned. Further, the compound (9) or the compound (10) itself may be used like a solvent.

With respect to the amount of fluorine to be used for the fluorination reaction, from the viewpoint of selectivity, the amount of fluorine atoms is preferably always an amount in excess equivalent to the amount of hydrogen atoms contained in the compound (9) from the beginning to the end of the reaction, and the amount of fluorine atoms is particularly preferably maintained to be at least 1.05 times by mol to the amount of hydrogen atoms.

Further, as the fluorine, a 100 volume% fluorine gas may be used as it is, or a mixed gas obtained by diluting the fluorine gas by an inert gas may be used. The inert gas may, for example, be nitrogen gas, argon gas or the like. The concentration of the fluorine gas in the mixed gas is preferably at least 10 volume%, particularly preferably at least 20 volume%.

The reaction temperature for the fluorination reaction is preferably at least -60°C and at most the boiling point of the compound (9), and when the reaction yield, selectivity and easiness of industrial operation are taken into consideration, from -50°C to +100°C is more preferred, and from -20°C to +50°C is further preferred. The reaction pressure is not particularly limited, and when the reaction yield, selectivity and easiness of industrial operation are taken into consideration, from normal presser to 2 MPa (gauge pressure, the same applies hereinafter) is preferred.

### [Step for obtaining the compound (11)]

The compound (11) can be produced from the compound (10) and is preferably produced by using, for example, the following method 1 or the following method 2.
Method 1: A method of conducting a decomposition reaction of the ester bond in the compound (10) to convert the "-CF₂OCOR^{f1}" group in the compound (10) to the " -COF" group thereby to prepare a compound of the formula (11) wherein X¹ is a fluorine atom (hereinafter referred to as the compound (11a).
Method 2: A method of subjecting the compound (10) and the compound represented by R-OH (R is the same as R when X¹ in the above formula (11) is -OR) to an ester exchange reaction to prepare a compound of the above formula (11) wherein X¹ is -OR (hereinafter referred to as the compound (11b).

The reaction schemes of the above method 1 and the above method 2 are shown by the following formulae.

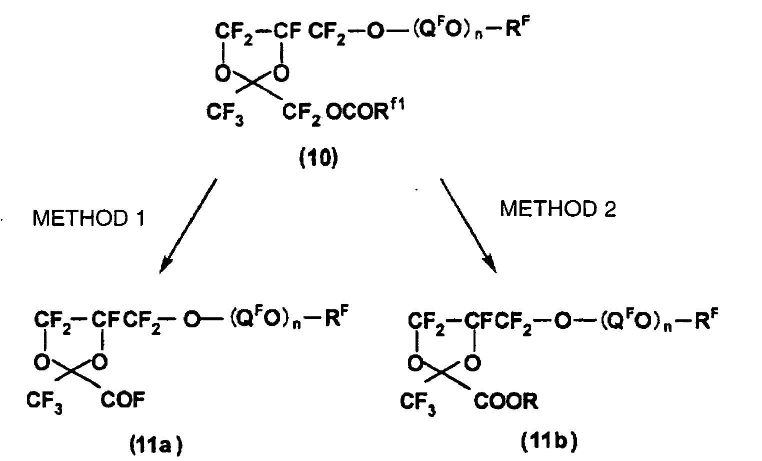

The method 1 can be conducted by using a known technique for decomposition reaction of ester bonds. For example, it is preferred to use a method of heating the compound (10) in a gas phase or a liquid phase, or a method of heating the compound (10) in the presence of a nucleophile or an electrophile. In each of the methods, the reaction temperature is preferably from 50° to 300°C, more preferably from 100 to 250°C,

In the method of heating in the presence of a nucleophile or an electrophile in the method 1, a solvent may or may not be used. It is preferred not to use a solvent since it is thereby possible to omit the step to separate the solvent. When a solvent is not used, the compound (10) can be used like a solvent.

It is preferred to use a nucleophile which can generate fluorine ions as the nucleophile in the method 1, since it is thereby possible to further lower the reaction temperature for the heat decomposition. When a nucleophile which can generate fluorine ions is used, the reaction temperature is preferably at least -30°C and at most the boiling point of the compound (11a). When the reaction is conducted around the boiling point of the compound (11a), the reaction is preferably conducted while withdrawing the product from the reaction system.

As the nucleophile which can generate fluorine ions, an alkali metal fluoride is preferably used. The alkali metal fluoride is preferably NaF, NaHF₂, KF or CsF. In the case where a decomposition reaction of ester bonds is conducted by using the alkali metal fluoride, it is considered that a - CF₂OCOR^{f1} group will be converted to a -COF group via a -CF₂OM group (M represents an alkali metal atom corresponding to the alkali metal fluoride used).

In the method 1, the compound (byproduct) represented by R^{f1}-COF is also produced along with the compound (11a). The compound (byproduct) can be reused as the compound (7) in the "steps until the compound (9) is obtained."

The method 2 can be conducted by using a known technique for ester exchange reaction.

The compound represented by R-OH in the method 2 may, for example, be methanol, ethanol, isopropanol or t-butanol. The reaction temperature for the ester exchange reaction is preferably at least -30°C and at most the boiling point of the compound represented by R-OH.

### [Step for obtaining the compound (1)]

The compound (1) is obtained, for example, by subjecting the compound (11) which is obtained by the above method 1 or method 2, to heat decomposition.

The method for heat decomposition is preferably the following method 3 or method 4.
Method 3: A method of heating the compound (11a) obtained by the above method 1 to let it undergo direct heat decomposition to prepare the compound (1).
Method 4: A method of reacting the compound (11b) and an alkali metal hydroxide to convert X¹ to a -OM' group (M' represents an alkali metal atom corresponding to the alkali metal hydroxide used), followed by heating for heat decomposition to prepare the compound (1).

The heat decomposition reaction in the method 3 may be conducted in either a gas or liquid phase, but it is effective and preferred to conduct it in a gas phase. In the case where the method 3 is conducted in a gas phase, a method is preferred wherein a tube reactor having glass beads, an alkali metal salt or an alkaline earth metal salt packed is prepared, and the compound (11a) in a gas state is passed thorough the tube reactor, and the produced gas containing the compound (1) is condensed and recovered. The compound (11a) is preferably passed thorough the tube reactor together with an inert gas. The reaction temperature for the reaction is preferably from 150° to 500°C, particularly preferably from 200 to 350°C,

The method 3 conducted in a gas phase is more suitable for an industrial production process than the method 4.

The alkali metal hydroxide in the method 4 is preferably NaOH or KOH. The amount of an alkali metal hydroxide to the amount of the compound (11b) is preferably from 0.95 to 1.05 mol, particularly preferably from 1.00 to 1.05 mol. The temperature for the reaction between the compound (11b) and the alkali metal hydroxide is preferably at least -30°C and at most the boiling point of the solvent. The reaction is preferably conducted in the presence of a solvent. The solvent may, for example, be methanol, ethanol, isopropanol or t-butanol.

The temperature for the heat decomposition is preferably from 150° to 400°C, particularly preferably from 150 to 300°C,

The method 4 is more advantageous for compounds having low stability to heat than the method 3 in that the reaction can be conducted at a low temperature.

The compound (1) obtained by the above methods is a novel fluorinated compound (fluorinated dioxolane compound). The compound (1) is excellent in physical properties such as heat resistance and transparency, and further, it is useful, for example, as a monomer to obtain a fluorinated polymer having adequate flexibility to avoid the cracking during the bulk polymerization.

Further, the absolute configuration of the asymmetric carbon atom in the compound (1) is not particularly limited, and it may be R-configuration or S-configuration. In the above methods for producing the fluorinated compound, usually, the absolute configuration of the asymmetric carbon atom is not maintained.

### <<Fluorinated polymer>>

The fluorinated polymer of the present invention is a polymer which comprises repeating units represented by the following formula (2): 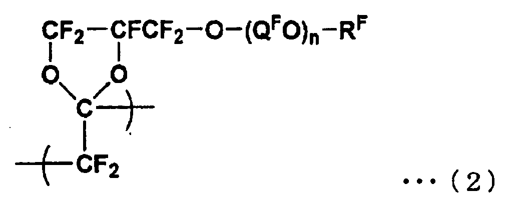 provided that, in the formula (2), Q^{F} is -CF₂-, -CF₂CF₂-, -CF₂CF₂CF₂-, -CF(CF₃)CF₂-, -CF₂CF(CF₃)- or -CF₂CF₂CF₂CF₂-, R^{F} is -CF₃, -CF₂CF₃, -CF₂CF₂CF₃, -CF(CF₃)₂, -CF₂CF₂CF₂CF₃, -CF₂CF(CF₃)₂, -CF(CF₃)CF₂CF₃, -(CF₂)₄CF₃ or -(CF₂)₅CF₃, and n is an integer of from 1 to 4.

In the present specification, the repeating units represented by the formula (2) are also represented by "repeating unit (2)". The repeating units represented by other formulae are also represented in the same manner.

The repeating unit (2) is a repeating unit based on the compound (1). The "repeating unit based on the compound (1)" means the repeating unit constructed by cleavage of the ethylenic double bound of the compound (1).

Q^{F}, R^{F} and n in the formula (2) are respectively the same as Q^{F}, R^{F} and n in the formula (1).

The fluorinated polymer of the present invention may contain repeating units based on another monomer which can be copolymerized with the repeating units (2) within a range not to impair the effect of the invention.

Such another monomer may, for example, be a radical polymerizable monomer.

The radical polymerizable monomer may be an α -olefin such as ethylene, propylene or isobutylene; a fluorinated olefin such as tetrafluoroethylene, hexafluoropropylene or chlorotrifluoroethylene; a perfluoro(alkyl vinyl ether) such as perfluoro(ethyl vinyl ether), perfluoro(propyl vinyl ether), perfluoro(hexyl vinyl ether), perfluoro(octyl vinyl ether), perfluoro(3,6-dioxa-5-methyl-1-decene) or perfluoro(3,6,9-trioxa-1-dodecene); a fluorinated cyclic monomer such as perfluoro(2,2-dimethyl-1,3-dioxole) or perfluoro(4-methoxy-1,3-dioxole); a cyclopolymerizable perfluorodiene such as perfluoro(allyl vinyl ether) or perfluoro(butenyl vinyl ether); an acryl ester such as methyl acrylate or methyl methacrylate; a vinyl ester such as vinyl acetate, vinyl benzoate or vinyl adamantate; a vinyl ether such as ethyl vinyl ether or cyclohexyl vinyl ether; a cyclic olefin such as cyclohexene, norbornene or norbornadiene; maleic anhydride, or vinyl chloride.

Further, a compound represented by the following formula (b1) (a perfluoro(2-methylene-1,3-dioxolane) having a substituent such as a perfluoro alkyl group) may also be mentioned: 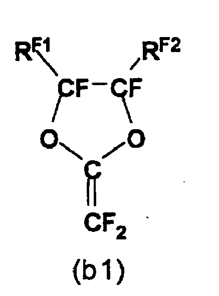 provided that, in the formula (b1), each of R^{F1} and R^{F2}, which are independent of each other, is a fluorine atom, a C₁₋₁₄ perfluoroalkyl group which may contain an etheric oxygen atom between carbon atoms, or a C₁₋₁₄ perfluoroalkoxy group which may contain an etheric oxygen atom between carbon atoms.

In the formula (b1), one of R^{F1} and R^{F2} is the perfluoroalkyl group, and the total number of carbon atoms in R^{F1} or R^{F2} is at least 4. Further, R^{F1} and R^{F2} may be bonded to each other, or may together form a C₄₋₁₄ perfluoroalkylene group which may contain an etheric oxygen atom.

Particularly preferably, R^{F1} is a fluorine atom and R^{F2} is a perfluoroalkyl group which may contain a C₄₋₁₄ etheric oxygen atom.

As the compound (b1), suitable ones are exemplified as follows: 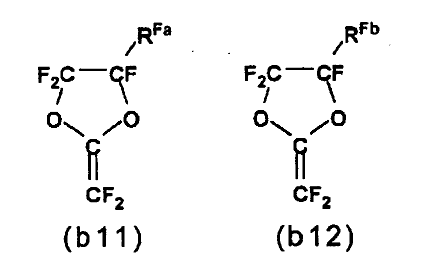 provided that R^{Fa} in the formula (b11) represents a C₄₋₁₄ perfluoroalkyl group and R^{Fb} in the compound (b12) represents a C₄₋₁₄ perfluoro(alkoxyalkyl) group.

As the compound (b11) represented by the formula (b11), suitable ones are exemplified as follows.

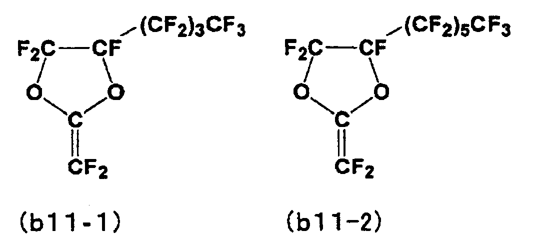

As the compound (b12) represented by the formula (b12), suitable ones are exemplified as follows.

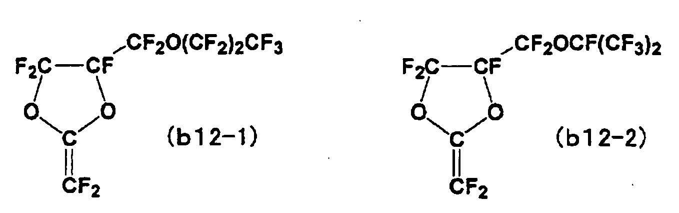

In a case where the fluorinated polymer of the present invention contains repeating units based on another monomer, said another monomer is preferably a fluorinated monomer.

The fluorinated monomer is more preferably at least one member selected from the group consisting of tetrafluoroethylene, hexafluoropropylene, chlorotrifluoroethylene, a perfluoro(alkyl vinyl ether), perfluoro(allyl vinyl ether), perfluoro(butenyl vinyl ether), perfluoro(2,2-dimethyl-1,3-dioxole), perfluoro(4-methoxy-1,3-dioxole), a perfluoro(3-alkyl-2-methylene-1,3-dioxolane) and a perfluoro(3-alkoxyalkyl-2-methylene-1,3-dioxolane).

The fluorinated monomer is more preferably a fluorinated cyclic monomer or cyclopolymerizable perfluorodiene.

The fluorinated cyclic monomer may be preferably perfluoro(2,2-dimethyl-1,3-dioxole), perfluoro(4-methoxy-1,3-dioxole), a perfluoro(3-alkyl-2-methylene-1,3-dioxolane), or a perfluoro(3-alkoxyalkyl-2-methylene-1,3-dioxolane). The perfluoro(3-alkyl-2-methylene-1,3-dioxolane) is more preferably perfluoro(3-butyl-2-methylene-1,3-dioxolane) or perfluoro(3-hexyl-2-methylene-1,3-dioxolane). Further, the perfluoro(3-alkoxyalkyl-2-methylene-1,3-dioxolane) is more preferably perfluoro(3-butoxymethyl-2-methylene-1,3-dioxolane) or perfluoro(3-isopropoxymethyl-2-methylene-1,3-dioxolane).

The cyclopolymerizable perfluorodiene is preferably perfluoro(allyl vinyl ether) or perfluoro(butenyl vinyl ether).

The proportion of repeating units (2) in the fluorinated polymer of the present invention can be optionally adjusted in accordance with the particular purpose of using the fluorinated polymer.

For example, the higher the proportion of the repeating units (2) in the fluorinated polymer is, the easier a fluorinated polymer which has a low glass transition temperature and high flexibility, is obtained.

The proportion of the repeating units (2) in the fluorinated polymer is preferably from 30 to 100 mass%, more preferably from 40 to 100 mass%, most preferably from 45 to 100 mass%. Within such a range, the fluorinated polymer tends to have a low glass transition temperature and to be excellent in flexibility.

Further, in the fluorinated polymer of the present invention, the repeating units (2) may preferably contain one type only or two or more types in combination.

The fluorinated polymer of the present invention is obtained by polymerizing only the compound (1) or copolymerizing a monomer containing the compound (1) in the presence of a polymerization initiating source.

The polymerization initiating source is not particularly limited so long as it is capable of letting the polymerization reaction proceed radically, and it may, for example, be a radical-generating agent, light or ionizing radiation. Among them, a radical-generating agent is preferred, and such a radical-generating agent may, for example, be a peroxide, an azo compound or a persulfate. Among them, a fluorinated organic peroxide is particularly preferred since the physical properties of the fluorinated polymer of the present invention is suitably maintained.

The polymerization method is not particularly limited, and it may, for example, be so-called bulk polymerization wherein a monomer is subjected to polymerization as it is; solution polymerization which is carried out in a fluorohydrocarbon, a chlorohydrocarbon, a fluorochlorohydrocarbon, an alcohol, a hydrocarbon or other organic solvent, which can dissolve the monomer; suspension polymerization which is carried out in an aqueous medium in the presence or absence of a suitable organic solvent; or emulsion polymerization which is carried out by adding an emulsifier to an aqueous medium.

The polymerization temperature is also not particularly limited, but it is preferred to optionally set it taking into consideration various factors such as the boiling point of the monomer, the required heating source, removal of the polymerization heat, etc. For example, suitable temperature setting can be carried out between 0 and 200°C, and practically suitable temperature setting can be carried out within a range of from room temperature to 100°C.

Further, the polymerization pressure may be a reduced pressure or an elevated pressure, and practically, the polymerization can properly be carried out within a range of from normal pressure to about 100 atm, preferably from normal pressure to about 10 atm.

### EXAMPLES

Now, the present invention will be described in further detail with reference to Examples. However, the present invention is by no means restricted thereto.

In the following Examples, 1,1,2-trichloro-1,2,2-trifluoroethane is referred to as "R-113", dichloropentafluoropropane is referred to as "R-225", gas chromatography is referred to as "GC" and gas chromatography mass spectrometry is referred to as "GC-MS".

A pressure is represented as an absolute pressure unless otherwise specified.

The purity of each compound was analyzed by GC method under the following conditions. GC purity represents the purity of the compound, which is obtained by a peak area ratio in GC analysis.

### Example 1: Example for production of the fluorinated compound (1-1)

The fluorinated compound (1-1) was produced by a series of production methods represented by the following steps (i) to (vi).

### Step (i)

To a flask (inner capacity: 500 mL), NaH (11 g) and tetrahydrofuran (150 mL) were added. Then, the interior of the flask was stirred under cooling with ice, and to the flask, CH₃CH₂(OCH₂CH₂)₂OH (30 g) was dropwise added over a period of 20 minutes. After completion of the dropwise addition, the interior of the flask was further stirred for 2 hours.

Then, to the flask, epichlorohydrin (100 g) was dropwise added over a period of 1 hour. After completion of the dropwise addition, the interior of the flask was stirred for 20 hours at a flask inner temperature of 25°C. Further, the interior of the flask was stirred for 2 hours at a flask inner temperature of 80°C.

Then, to the flask, acetic acid was added until the pH of the solution in the flask became 7, and the formed salt was removed by filtration to obtain a reaction crude liquid, followed by distillation under reduced pressure to obtain the following compound (5-1) (GC purity: 99 %) as a fraction (28 g) of from 95 to 97°C/2hPa.

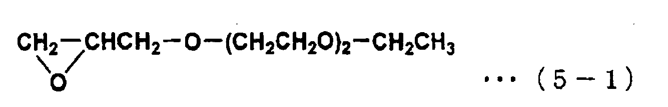

### Step (ii)

To a flask (inner capacity: 200 mL) under nitrogen gas atmosphere, BF₃O(CH₂CH₃)₂ (1 g) and acetone (46 g) were added. Then, the compound (5-1) (30 g) was added to the flask under cooling with ice, and then, the interior of the flask was stirred for 2 hours at a flask inner temperature of 25°C.

Then, CH₃COCH₂OCOCF(CF₃) O(CF₂)₂CF₃ (61 g) was added thereto, and then, acetone as a low boiling point component was distilled off under a flask inner pressure of 1 hPa at a flask inner temperature of 30°C. The solution in the flask was washed by a saturated sodium bicarbonate aqueous solution, then purified by silica gel chromatography and analyzed by NMR to confirm the formation of the following compound (9-1) (6 g) (GC purity: 95 %).

NMR data of the compound (9-1) were as follows:

¹H-NMR (282.7 MHz, solvent: CDCl₃, standard: CFCl₃) δ (ppm): 1.2 (3H), 1.4(3H) and from 3.5 to 4.5 (17H).

¹⁹F-NMR (282.7 MHz, solvent: CDCl₃, standard: CFCl₃) δ (ppm): -80.1 (1 F), -81.7 (3F), -82.4 (3F), -86.5 (1 F), -130.1 (2F) and -132.2 (1 F).

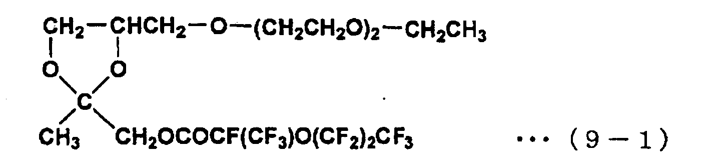

### Step (iii)

To an autoclave (inner capacity: 500 mL, made of nickel), R-113 (314 g) was added, followed by stirring to maintain the autoclave inner temperature at 25°C. At the autoclave gas outlet, a cooler kept at 20°C, a NaF pellet-packed bed and a cooler kept at -10°C were set in series. Further, to the cooler kept at -10°C, a liquid-returning line was set to return a condensed liquid to the autoclave.

Then, at 25°C, nitrogen gas was blown into the autoclave for 1 hour, and then, a fluorine gas diluted to 20 vol% with nitrogen gas (hereinafter referred to as a 20% fluorine gas) was blown therein at a flow rate of 0.1615 mol/h for 1 hour. Then, while blowing the 20% fluorine gas therein at the same flow rate, a solution having the compound (9-1) (6 g) dissolved in R-113 (95 g) was injected in the autoclave over a period of 4.2 hours.

Then, while blowing the 20% fluorine gas therein at the same flow rate, the autoclave inner pressure was raised to 0.10 MPa (gage pressure). Then, while increasing the autoclave inner temperature from 25°C to 40°C, a R-113 solution (9 mL) containing benzene at a concentration of 0.01 g/mL was injected into the autoclave, followed by stirring the interior of the autoclave. This operation was repeated 3 times. The total injected amount of benzene was 0.2 g, and the total injected amount of R-113 was 21 mL.

The autoclave inner pressure was adjusted to atmospheric pressure, and nitrogen gas was blown in the autoclave for 1 hour, and the content in the autoclave was collected and analyzed by ¹⁹F-NMR and GC to confirm the formation of the following compound (10-1) (yield: 90 %).

¹⁹F-NMR data of the compound (10-1) were as follows:

¹⁹F-NMR (282.7 MHz, solvent: CDCl₃, standard: CFCl₃) δ (ppm): -77.5 (1 F), -80.5 (4F), from -82.0 to -83.5 (11 F), -84.0 (1 F), from -86.0 to -88.0 (5F), -89.0 (8F), -122.0 (1 F), -130.0 (2F) and -132.0 (1 F).

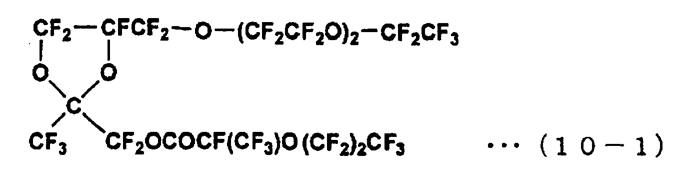

### Step (iv)

To a bottle (100mL, made of polyethylene), a R-113 solution (44 g) containing the compound (10-1) was added. While the interior of the bottle was stirred under cooling with ice, methanol (0.83 g) and R-225 (10 mL) were dropwise added to the bottle over a period of 5 minutes. After completion of the dropwise addition, the interior of the bottle was stirred for 5 hours at 25°C. Then, a low boiling point component was distilled off from the content in the bottle to obtain the following compound (11-1) (6 g) (GC purity: 78 %).

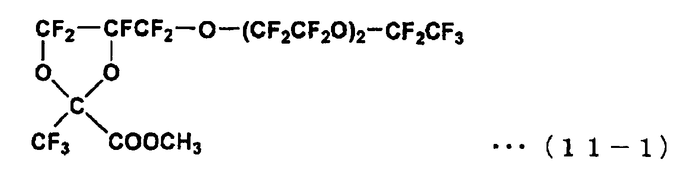

### Step (v)

To a flask (inner capacity: 100 mL), the compound (11-1) (6 g) and methanol (10 mL) were added, and the interior of the flask was stirred under cooling with ice. Then, to the flask, a 20 mass % KOH aqueous solution (4 g) was dropwise added over a period of 5 minutes. After completion of the dropwise addition, the interior of the flask was stirred for 18 hours at 25°C. Then, the content in the flask was distilled off under reduced pressure, followed by drying under reduced pressure at 80°C for 20 hours to obtain the following compound (11-1)' (5 g) which was slightly yellow crystal.

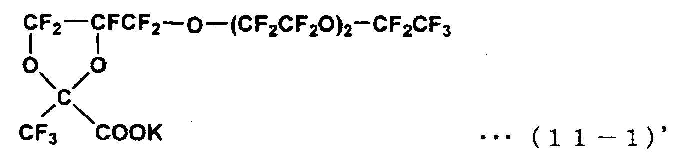

### Step (vi)

To a flask (inner capacity: 20 mL), the compound (11-1)' (5 g) was added, and while the interior of the flask was evacuated, the flask was heated at from 250°C to 260°C for heat decomposition. In the flask, a trop tube of -78°C was set to collect generated gas. The liquid (3 g) collected under heating in the trop tube was analyzed by ¹⁹F-NMR and GC to confirm the formation of the following fluorinated compound (1-1). Then, the liquid was distilled to obtain the fluorinated compound (1-1) (2 g) (GC purity: 98 %) as a fraction of from 50°C to 55°C/9hPa.

GC-MS data and ¹⁹F-NMR data of the compound (1-1) were as follows:

Molecular weight [mass number (M) / valence of an ion (e)] = 592

¹⁹F-NMR (282.7 MHz, solvent: CDCl₃, standard: CFCl₃) δ (ppm): from -82.4 to -84.5 (3F), -87.4 (3F), from -88.1 to -88.7 (1 F), from -89.0 to -89.5 (10F), -125.4 (1 F), -126.8 (1 F) and -128.8 (1 F).

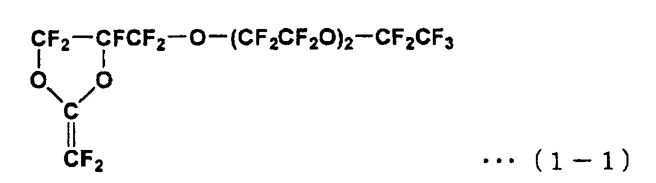

### Example 2: Example for homo polymerization of the fluorinated compound (1-1)

To a vial (made of glass), the compound (1-1) (1 g) and perfluorobenzoyl peroxide were added, and the space in the vial was replaced by nitrogen gas, followed by sealing the vial.

Then, the vial was heated at 60°C for 15 hours and further heated at 200°C for 1 hour to remove a volatile component in the vial. Then, the vial was cooled to obtain a colorless and transparent fluorinated polymer (0.96 g) which was rubbery at 25°C.

The glass transition temperature of the fluorinated polymer measured by a differential scanning calorimeter was 10°C.

### Example 3: Example for copolymerization of the fluorinated compound (1-1) with another monomer

To a vial (made of glass), the compound (1-1) (0.5 g), the following compound (b11-2) (0.5 g) and perfluorobenzoyl peroxide (2 mg) were added, and the space in the vial was replaced by nitrogen gas, followed by sealing the vial.

Then, the vial was heated at 60°C for 15 hours and further heated at 200°C for 1 hour to remove a volatile component in the vial. Then, the vial was cooled to obtain a colorless and transparent fluorinated polymer (0.98 g).

The glass transition temperature of the fluorinated polymer measured by a differential scanning calorimeter was 45°C.

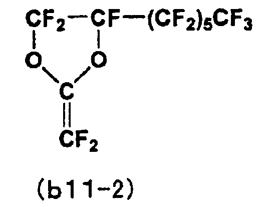

### Example 4: Example for production of the fluorinated compound (1-2)

The fluorinated compound (1-1) was produced by a series of production methods represented by the following steps from (i) to (vii).

### Step (i)

To a flask (inner capacity: 1,000 mL), NaH (13 g) and tetrahydrofuran (400 mL) were added. Then, the interior of the flask was stirred under cooling with ice, and, to the flask, CH₃CH₂CH₂CH₂OCH₂CH₂OH (50 g) was dropwise added over a period of 20 minutes. After completion of the dropwise addition, the interior of the flask was stirred for 1 hour at room temperature.

Then, to the flask, epichlorohydrin (90 g) was dropwise added over a period of 20 minutes. After completion of the dropwise addition, the interior of the flask was stirred for 3 days at a flask inner temperature of 25°C. Further, the interior of the flask was stirred for 2.5 hours at a flask inner temperature of 80°C.

Then, to the flask, acetic acid was added until the pH of the solution in the flask became 7, and a liquid separating operation by water and AK-225 was repeated 5 times. The obtained solution was distilled under reduced pressure, and the following compound (5-2) was obtained (GC purity: 98 %) as a fraction (45 g) of from 72 to 75°C/5hPa. Then, by the same method, the synthesis was further repeated twice to obtain the total 154 g of the compound (5-2).

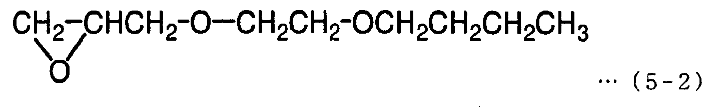

### Step (ii)

To a flask (inner capacity: 300 mL) under nitrogen gas atmosphere, BF₃O(CH₂CH₃)₂ (2.7 g) and acetone (100 mL) were added. Then, the compound (5-2) (30 g) was added to the flask under cooling with ice. Then, the interior of the flask was stirred for 18 hours at a flask inner temperature of 25°C.

Then, hydroxy acetone (26 g) was added thereto, and then, acetone as a low boiling point component was distilled off under a flask inner pressure of 150 hPa at a flask inner temperature of 40°C. The solution in the flask was washed by a saturated sodium bicarbonate aqueous solution and a 1 N hydrochloric acid aqueous solution, and purified by silica gel chromatography and analyzed by NMR to confirm the formation of the following compound (12-2) (20 g) (GC purity: 94 %). Then, a scale up synthesis was conducted in the same manner to obtain 80 g of the compound (12-2) from 120 g of a raw material (5-2).

NMR data of the compound (12-2) were as follows:
¹H-NMR (282.7 MHz, solvent: CDCl₃, standard: CFCl₃) δ (ppm): 0.9 (3H), from 1.3 to 1.4 (5H), 1.5(2H), 2.1 (1H), from 3.4 to 3.7 (10H), from 3.8 to 4.0 (1H) and from 4.1 to 4.4 (2H).

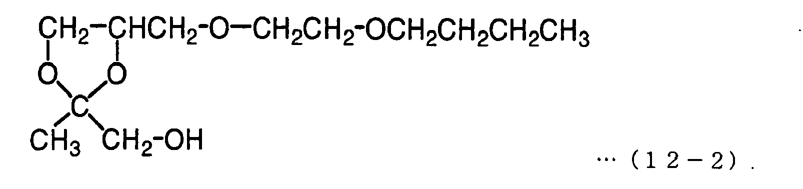

### Step (iii)

To a flask (inner capacity: 1,000 mL), the compound (12-2) (97 g), sodium fluoride (44 g) and AK-225 (300 mL) were added and stirred. CF₃CF₂CF₂OCF(CF₃)CF₂OCF(CH₃)COF (214 g) was dropwise added to the flask under cooling with ice over a period of 30 minutes. Then, the flask was stirred for 18 hours at room temperature. Sodium fluoride in the flask was removed by filtration, and the filtered solution was washed twice by a saturated sodium bicarbonate aqueous solution. The obtained solution was distilled under reduced pressure to obtain the following compound (9-2) (GC purity: 100 %) as a fraction (230 g) of 80°C/2 mmHg.

NMR data of the compound (9-2) were as follows:
¹H-NMR (282.7 MHz, solvent: CDCl₃, standard: CFCl₃) δ (ppm): 0.9 (3H), from 1.3 to 1.5 (5H), 1.56 (1H), from 3.4 to 3.7 (8H), from 3.7 to 3.9 (1H) and from 4.0 to 4.6 (4H).
¹⁹F-NMR (282.7 MHz, solvent: CDCl₃, standard: CFCl₃) δ (ppm): from -79 to -80 (1 F), -80.5 (3F), from -81.7 to -82.1 (5F), -82.5 (3F), from -84.2 to -85.5 (1 F), from -130.1 to -130.6 (2F), -132.1 (1 F) and -145.6 (1 F).

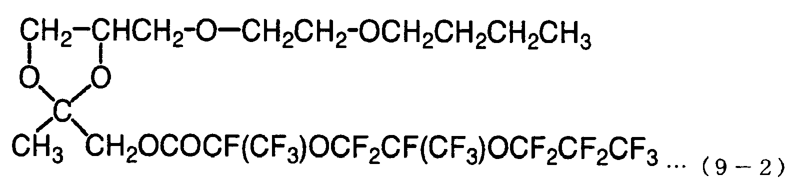

### Step (iv)

To an autoclave (inner capacity: 500 mL, made of nickel), R-113 (313 g) was added, followed by stirring to maintain the autoclave inner temperature at 25°C. At the autoclave gas outlet, a cooler kept at 20°C, a NaF pellet-packed bed and a cooler kept at -10°C were set in series. Further, to the cooler kept at -10°C, a liquid-returning line was set to return a condensed liquid to the autoclave.

Then, at 25°C, nitrogen gas was blown into the autoclave for 1 hour, and then, a fluorine gas diluted to 20 vol% with nitrogen gas (hereinafter referred to as a 20% fluorine gas) was blown therein at a flow rate of 0.08892 mol/h for 1 hour. Then, while blowing the 20% fluorine gas therein at the same flow rate, a solution having the compound (9-2) (5 g) dissolved in R-113 (45 g) was injected in the autoclave over a period of 2.7 hours.

Then, while blowing the 20% fluorine gas therein at the same flow rate, the autoclave inner pressure was raised to 0.10 MPa (gage pressure). Then, while increasing the autoclave inner temperature from 25°C to 40°C, a R-113 solution (6 mL) containing benzene at a concentration of 0.01 g/mL was injected into the autoclave, followed by stirring the interior of the autoclave. This operation was repeated 3 times. The total injected amount of benzene was 0.2 g, and the total injected amount of R-113 was 21 mL.

The autoclave inner pressure was adjusted to atmospheric pressure, and nitrogen gas was blown in the autoclave for 1 hour, and the content in the autoclave was collected and analyzed by ¹⁹F-NMR and GC to confirm the formation of the following compound (10-2) (yield: 89 %). Then, by using a 3L autoclave, a scale up synthesis was conducted twice in the same manner to obtain a crude product containing 320 g of the compound (10-2) from 230 g of a raw material.

¹⁹F-NMR data of the compound (10-2) were as follows:

¹⁹F-NMR (282.7 MHz, solvent: CDCl₃, standard: CFCl₃) δ (ppm): -77.5 (1F), from -80.5 to -83.5 (20F), from -84.0 to -87.5 (6F), -89 (4F), -122 (1 F), -127 (4F), -130 (2F), -132 (1 F) and -145 (1 F).

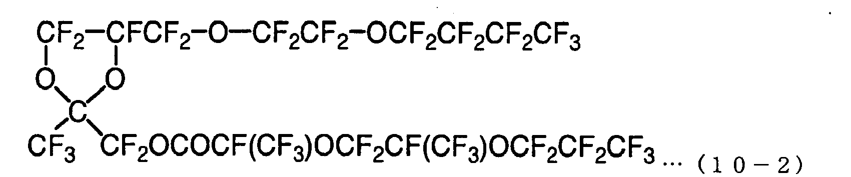

### Step (v)

To a flask (inner capacity: 500 mL), 161 g (GC purity: 89 %) of the compound (10-2) obtained in the step (iv), AK-225 (200 mL) and sodium fluoride (48 g) were added. The interior of the flask was stirred under cooling with ice, and methanol (40.4 g) and R-225 (86 mL) were dropwise added thereto over a period of 20 minutes. After completion of the dropwise addition, the interior of the bottle was stirred for 3 days at 25°C. Then, the obtained solution was filtered to remove sodium fluoride, and the solution was distilled off. A distillation was conducted to obtain the following compound (11-2) (40 g) (GC purity: 93 %) as a fraction of from 96 to 99°C/23hPa. Similar reactions were repeated to obtain the total 80 g of the compound (11-2).

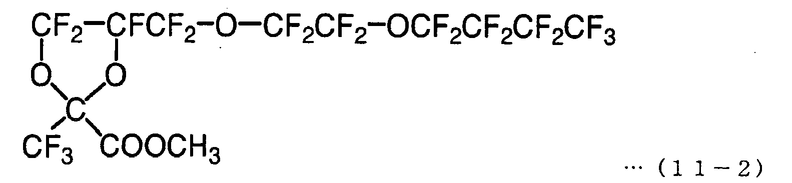

### Step (vi)

To a flask (inner capacity: 300 mL), the compound (11-2) (75 g) and methanol (65 mL) were added, and the interior of the flask was stirred under cooling with ice. Then, to the flask, a 20 mass% potassium hydroxide methanol solution (35 g) was dropwise added over a period of 12 minutes. After completion of the dropwise addition, the interior of the flask was stirred for 2 days at a flask inner temperature of 25°C. Then, the content in the flask was distilled off under reduced pressure, followed by drying under reduced pressure at 80°C for 20 hours to obtain the following compound (11-2)' (68 g) which was slightly yellow crystal.

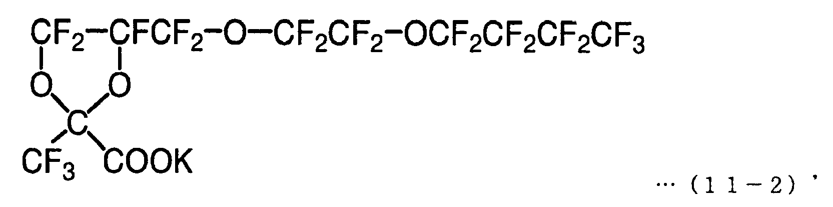

### Step (vii)

To a flask (inner capacity: 200 mL), the compound (11-2)' (48 g) was added, and while the interior of the flask was evacuated, the flask was heated at from 260°C to 280°C for heat decomposition. In the flask, a trop tube of -78°C to collect generated gas and a tarp tube cooled by liquid nitrogen were set in series. As a result of analyzing a liquid (41 g) collected under heating in the trop tube by ¹⁹F-NMR and GC, the formation of the following fluorinated compound (1-1) was confirmed. Then, the liquid was dilstilled to obtain the fluorinated compound (1-2) (32 g) (GC purity: 95 %) as a fraction of from 42°C to 44°C/8hPa to 9hPa.

¹⁹F-NMR data of the compound (1-1) were as follows:

¹⁹F-NMR (282.7 MHz, solvent: CDCl₃, standard: CFCl₃) δ (ppm): -81.8 (3F), from -82.5 to -83.5 (2F), from -83.9 to -84.3 (3F), from -88.0 to -88.6 (1 F), -89.3 (4F), from -125.3 to -125.8 (1 F), from -126.7 to -127.3 (5F) and -128.8 (1 F).

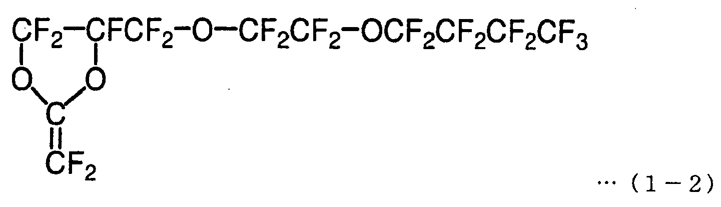

### Example 5: Example for homo polymerization of the fluorinated compound (1-2)

To a vial (made of glass), the compound (1-2) (0.43 g) and perfluorobenzoyl peroxide (1.5 mg) were added, and the space in the vial was replaced by nitrogen gas, followed by sealing the vial.

Then, the vial was heated at 60°C for 1 hour, at 70°C for 19 hours, at 90°C for 48 hours and further heated at 120°C for 3 hours. Further, at 100°C under vacuum condition, a volatile component in the vial was removed. Then, the vial was cooled to obtain a colorless and transparent fluorinated polymer (0.41 g) which was rubbery at 25°C.

The glass transition temperature of the fluorinated polymer measured by a differential scanning calorimeter was 27°C.

### Example 6: Example for copolymerization of the fluorinated compound (1-2) with another monomer

To a vial (made of glass), the compound (1-2) (0.22g), the compound (b11-2) (0.23g) and perfluorobenzoyl peroxide (1.7 mg) were added, and the space in the vial was replaced by nitrogen gas, followed by sealing the vial.

Then, the vial was heated at 60°C for 2 hours, at 70°C for 20 hours, at 90°C for 4 hours, at 120°C for 2 hours, at 150°C for 1.5 hours and further heated at 100°C for 1 hour under vacuum condition to remove a volatile component in the vial. Then, the vial was cooled to obtain a colorless and transparent fluorinated polymer (0.43 g).

The glass transition temperature of the fluorinated polymer measured by a differential scanning calorimeter was 59.0°C.

### Example 7: Example for production of the fluorinated compound (1-3)

The fluorinated compound (1-1) was produced by a series of production methods represented by the following steps from (i) to (v).

### Step (i)

To a flask (inner capacity: 1,000 mL), the following compound (1-3) (94 g), sodium fluoride (48 g) and AK-225 (193 mL) were added and stirred. CF₃CF₂CF₂OCF(CF₃)CF₂OCF(CF₃) COF (239 g) was dropwise added to the flask over a period of 30 minutes under cooling with ice. Then, the flask was stirred for 5 days at room temperature. The sodium fluoride in the flask was removed by filtration and the filtered solution was washed once by a saturated sodium bicarbonate aqueous solution. The obtained solution was distilled under reduced pressure to obtain the following compound (9-3) (GC purity: 100 %) as a fraction (199 g) of from 115 to 118°C/1 mmHg.

NMR data of the compound (9-3) were as follows:
¹H-NMR (282.7 MHz, solvent: CDCl₃, standard: TMS) δ (ppm): 0.9 (6H), 1.4 (3H), 1.85 (1H), from 3.5 to 3.7 (6H), from 3.7 to 3.9 (1H) and from 4.0 to 4.6 (4H).
¹⁹F-NMR (282.7 MHz, solvent: CDCl₃, standard: CFCl₃) δ (ppm): from -79 to -80 (1 F), -80.5 (3F), from -81.7 to -82.1 (5F), -82.5 (3F), from -84.2 to -85.5 (1 F), from -130.1 to -130.6 (2F), -132.1 (1F) and -145.6 (1F).

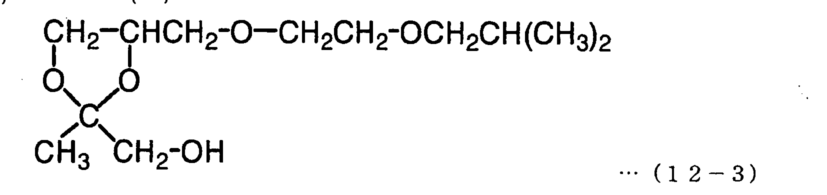

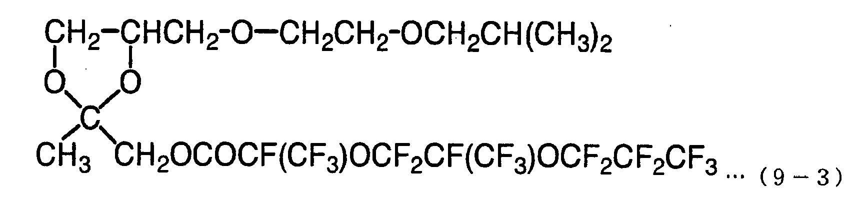

### Step (ii)

To an autoclave (inner capacity: 500 mL, made of nickel), R-113 (313 g) was added, followed by stirring to maintain the autoclave inner temperature at 25°C. At the autoclave gas outlet, a cooler kept at 20°C, a NaF pellet-packed bed and a cooler kept at -10°C were set in series. Further, to the cooler kept at -10°C, a liquid-returning line was set to return a condensed liquid to the autoclave.

Then, at 25°C, nitrogen gas was blown into the autoclave for 1 hour, and then, a fluorine gas diluted to 20 vol% with nitrogen gas (hereinafter referred to as a 20% fluorine gas) was blown therein at a flow rate of 0.17393 mol/h for 1 hour. Then, while blowing the 20% fluorine gas therein at the same flow rate, a solution having the compound (9-3) (10 g) dissolved in R-113 (97 g) was injected in the autoclave over a period of 2.9 hours.

Then, while blowing the 20% fluorine gas therein at the same flow rate, the autoclave inner pressure was raised to 0.10 MPa (gage pressure). Then, while increasing the autoclave inner temperature from 25°C to 40°C, a R-113 solution (6 mL) containing benzene at a concentration of 0.01 g/mL was injected into the autoclave, followed by stirring the interior of the autoclave. This operation was repeated 3 times. The total injected amount of benzene was 0.2 g, and the total injected amount of R-113 was 21 mL.

The autoclave inner pressure was adjusted to atmospheric pressure and nitrogen gas was blown in the autoclave for 1 hour, and then, the content in the autoclave was collected and analyzed by ¹⁹F-NMR and GC to comfirm the formation of the following compound (10-3) (yield: 85 %). In the same manner, a scale up synthesis was conducted, and the reaction of the raw material (150 g) was conducted to confirm the formation of the compound (10-3) (yield: 85 %) by ¹⁹F-NMR and GC.

¹⁹F-NMR data of the compound (10-3) were as follows:
¹⁹F-NMR (282.7 MHz, solvent: CDCl₃, standard: CFCl₃) δ (ppm): -74 (6F), from -77.5 to -87.5 (24F), -89 (4F), -122 (1 F), -130 (2F), -132 (1 F). -145 (1 F) and -188 (1 F).

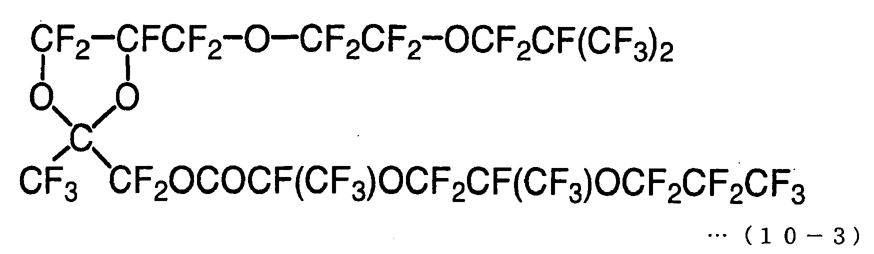

### Step (iii)

To a flask (inner capacity: 500 mL), a R-113 solution (133 g, GC purity: 93 %) containing the compound (10-3), AK-225 (124 mL) and sodium fluoride (41 g) were added. While the interior of the bottle was stirred under cooling with ice, methanol (34.7 g) was dropwise added thereto over a period of about 30 minutes. After completion of the dropwise addition, the interior of the bottle was stirred for 25 hours at 25°C. Then, the obtained solution was filtered to remove the sodium fluoride, and the solution was distilled off. Distillation was conducted to obtain the following compound (11-3) (59 g)(GC purity: 89 %) as a fraction of from 55 to 57°C/4hPa.

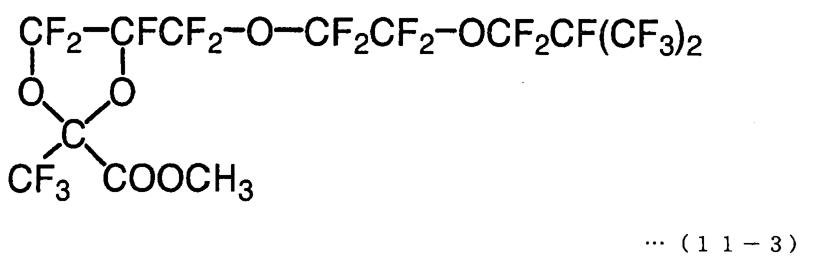

### Step (iv)

To a flask (inner capacity: 300 mL), the compound (11-3) (59 g, GC purity: 89 %) and methanol (53 mL) were added, and the interior of the flask was stirred under cooling with ice. Then, to the flask, a 20 mass% potassium hydroxide methanol solution (26 g) was dropwise added over a period of 3 minutes. After completion of the dropwise addition, the interior of the flask was stirred for 2 days at a flask inner temperature of 25°C. Then, the content in the flask was distilled off under reduced pressure, followed by drying under reduced pressure at 80°C for 20 hours to obtain the following compound (11-3)' (60 g) which was slightly yellow crystal.

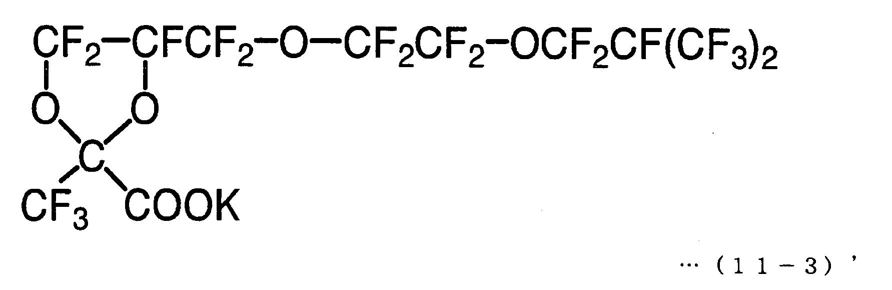

### Step (v)

To a flask (inner capacity: 200 mL), the compound (11-3)' (60 g) was added, and while the interior of the flask was evacuated, the flask was heated at from 260°C to 280°C for heat decomposition. In the flask, a trop tube of -78°C to collect generated gas and a trop tube cooled by liquid nitrogen were set in series. As a result of analyzing a liquid (43 g) collected under heating in the trop tube by ¹⁹F-NMR and GC, the formmation of the following fluorinated compound (1-3)) was confirmed. Then, the liquid was distilled to obtain the fluorinated compound (1-3) (30 g) (GC purity: 96 %) as a fraction of from 42°C to 44°C/8hPa to 9hPa.

¹⁹F-NMR data of the compound (1-3) were as follows:

¹⁹F-NMR (282.7 MHz, solvent: CDCl₃, standard: CFCl₃) δ (ppm): -74 (6F), -77.5 (2F), from -82.6 to -84.6 (3F), from -88.0 to -88.6 (1 F), -89.5 (4F), from -125.4 to -125.9 (1 F), from -126.7 to -127.5 (1 F), -128.7 (1 F) and -188 (1 F).

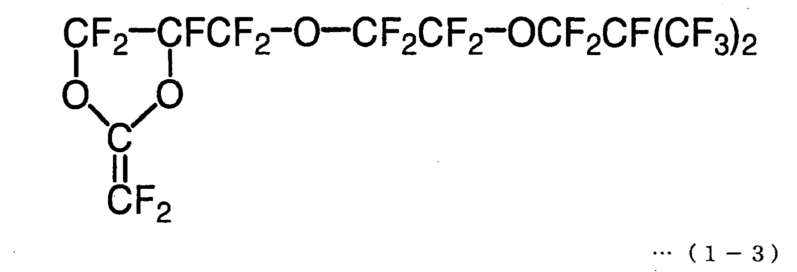

### Example 8: Example for homo polymerization of the fluorinated compound (1-3)

To a vial (made of glass), the compound (1-3) (0.45 g) and perfluorobenzoyl peroxide (1.4 mg) were added, and the space in the vial was replaced by nitrogen gas, followed by sealing the vial.

Then, the vial was heated at 60°C for 1 hour, at 70°C for 19 hours, at 90°C for 48 hours and further heated at 120°C for 3 hours. Further, at 100°C under vacuum condition, a volatile component in the vial was removed. Then, the vial was cooled to obtain a colorless and transparent fluorinated polymer (0.43 g) which was rubbery at 25°C.

The glass transition temperature of the fluorinated polymer measured by a differential scanning calorimeter was 35°C.

From the foregoing, it was confirmed that the homopolymers comprising repeating units based on the fluorinated compounds (1-1) to (1-3) and copolymers containing such repeating units of the present invention have high transparency since they are colorless and transparent, and they have low glass transition temperatures and adequate flexibility. The homopolymers and the copolymers have good heat resistance since they are fluorinated polymers.

Therefore, the present invention can provide a fluorinated polymer having high transparency, good heat resistance and adequate flexibility.

### INDUSTRIAL APPLICABILITY

The fluorinated polymer of the present invention has high transparency, good heat resistance and adequate flexibility, for example, to avoid the cracking during the bulk polymerization. Further, the fluorinated polymer of the present invention is excellent in light resistance (particularly, light resistance to the short wavelength light of from 200 to 500 nm).

Therefore, the fluorinated polymer of the present invention is useful for an optical material. The optical material may be used for an application to a core material or clad material of optical fiber, a core material or clad material of an optical waveguide, a pellicle material, a surface protecting material for a display (e.g. PDP, LCD, FED or an organic EL), a surface protecting material for a lens (e.g. a condensing lens for a light-emitting device, an artificial crystalline lens, a contact lens or a low refractive index lens), a material for a lens (e.g. a condensing lens for a light-emitting device, an artificial crystalline lens, a contact lens or a low refractive index lens), or a sealing material for a device (e.g. a light-emitting device, a solar cell device or a semiconductor device).

As other applications, it can be used for e.g. various sealers or sealing materials required to have chemical resistance, weather resistance, heat resistance, moisture resistance, oil resistance or the like.

The entire disclosure of Japanese Patent Application No. 2006-343518 filed on December 20, 2006 including specifications, claims and summaries is incorporated herein by reference in its entirety.

## Claims

1. A fluorinated compound represented by the following formula (1): 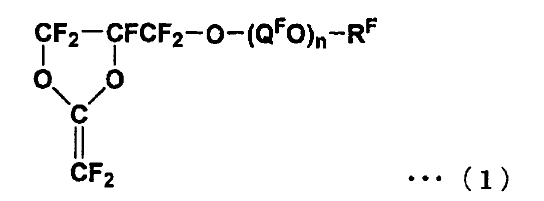 provided that, in the formula (1), Q^{F} is -CF₂-, -CF₂CF₂-, -CF₂CF₂CF₂-, -CF(CF₃)CF₂-, -CF₂CF(CF₃)- or -CF₂CF₂CF₂CF₂-, R^{F} is -CF₃, -CF₂CF₃, -CF₂CF₂CF₃, -CF(CF₃)₂, -CF₂CF₂CF₂CF₃, -CF₂CF(CF₃)₂, -CF(CF₃)CF₂CF₃, -(CF₂)₄CF₃ or -(CF₂)₅CF₃, and n is an integer of from 1 to 4.

2. The fluorinated compound according to Claim 1, wherein Q^{F} in the above formula (1) is -CF₂CF₂-, -CF₂CF₂CF₂- or -CF₂CF₂CF₂CF₂-.

3. The fluorinated compound according to Claim 1 or 2, wherein n in the above formula (1) is 1 or 2.

4. A fluorinated compound represented by one of the following formulae:

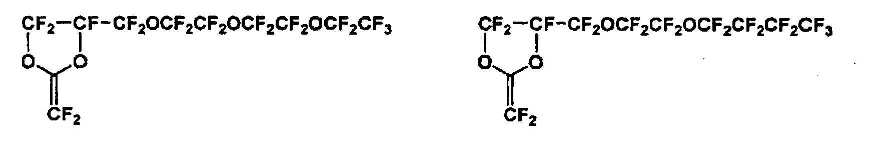

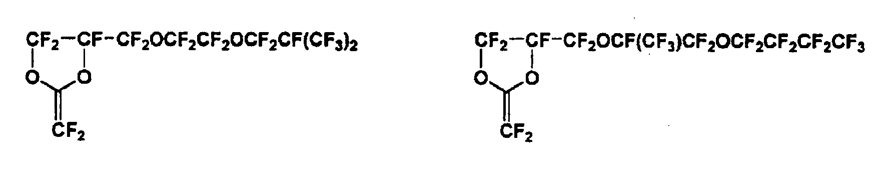

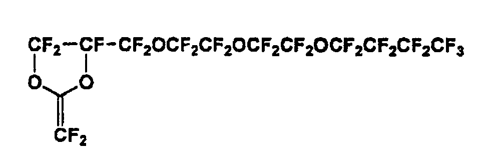

5. A fluorinated polymer comprising repeating units represented by the following formula (2): 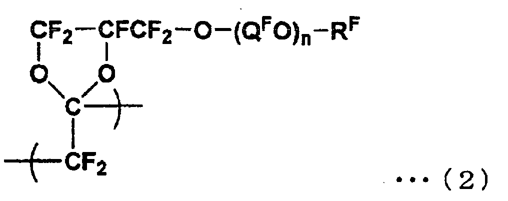 provided that, in the formula (2), Q^{F} is -CF₂-, -CF₂CF₂-, -CF₂CF₂CF₂-, -CF(CF₃)CF₂-, -CF₂CF(CF₃)- or -CF₂CF₂CF₂CF₂-, R^{F} is -CF₃, -CF₂CF₃, -CF₂CF₂CF₃, -CF(CF₃)₂, -CF₂CF₂CF₂CF₃, -CF₂CF(CF₃)₂, -CF(CF₃)CF₂CF₃, -(CF₂)₄CF₃ or -(CF₂)₅CF₃, and n is an integer of from 1 to 4.

6. The fluorinated polymer according to Claim 5, wherein Q^{F} in the above formula (2) is -CF₂CF₂-, -CF₂CF₂CF₂- or -CF₂CF₂CF₂CF₂-.

7. The fluorinated polymer according to Claim 5 or 6, wherein n in the above formula (2) is 1 or 2.

8. The fluorinated polymer according to any one of Claims 5 to 7, which contains from 30 to 100 mass% of repeating units represented by the above formula (2).

9. The fluorinated polymer according to any one of Claims 5 to 8, which contains repeating units based on another monomer, and said another monomer is a fluorinated monomer.

10. The fluorinated polymer according to Claim 9, wherein the fluorinated monomer is at least one selected from the group consisting of tetrafluoroethylene, hexafluoropropylene, chlorotrifluoroethylene, a perfluoro(alkyl vinyl ether), perfluoro(allyl vinyl ether), perfluoro(butenyl vinyl ether), perfluoro(2,2-dimethyl-1,3-dioxole), perfluoro(4-methoxy-1,3-dioxole), a perfluoro(3-alkyl-2-methylene-1,3-dioxolane) and a perfluoro(3-alkoxyalkyl-2-methylene-1,3-dioxolane).
